# EUROPEAN PATENT APPLICATION

(11) **EP 4 681 652 A1**
(43) Date of publication of application: **21.01.2026**
(21) Application number: 25189261.8
(22) Date of filing: 14.07.2025
(51) Int. Cl.: A61B 6/51, A61B 6/02, A61B 6/00

(54) **DENTAL X-RAY RADIOGRAPHY APPARATUS**

(30) Priority: 15.07.2024 KR 20240092799
(71) Applicant: Vatech Co., Ltd., Hwaseong-si, Gyeonggi-do 18449 (KR); Vatech Ewoo Holdings Co., Ltd., Hwaseong-si, Gyeonggi-do 18449 (KR)
(72) Inventor: LIM, Sung Hwan, Hwaseong-si (KR); CHOI, Jeong Bong, Hwaseong-si (KR); KIM, You Suk, Hwaseong-si (KR)
(74) Representative: Lorenz & Kollegen

(57) **Abstract**

Proposed is an X-ray radiography apparatus. The X-ray radiography apparatus includes a radiography part including an X-ray generator and an X-ray sensor facing each other with a radiography target therebetween, a first driving part configured to raise or lower the radiography part from a first position to a second position, and a second driving part configured to rotate the X-ray generator and the X-ray sensor while the X-ray generator and the X-ray sensor face each other, wherein the second driving part is configured to sequentially increase a rotation speed of the X-ray generator and the X-ray sensor, maintain the rotation speed at a constant speed, and decrease the rotation speed when the radiography part is raised or lowered from the first position to the second position by the first driving part.

## Description

### CROSS REFERENCE TO RELATED APPLICATION

The present application claims priority to Korean Patent Application No. 10-2024-0092799, filed 15 July 2024, the entire contents of which is incorporated herein for all purposes by this reference.

### BACKGROUND OF THE INVENTION

### Field of the Invention

The present disclosure relates to a dental X-ray radiography apparatus and a method of operating the same.

### Description of the Related Art

X-rays are a type of electromagnetic wave having a wavelength approximately 1/1000 that of visible light, and exhibit characteristics of rectilinear propagation, penetrability, and attenuation depending on the X-ray attenuation coefficient of a material located in the path of propagation. An X-ray image is a radiography image using such inherent characteristics of X-rays, and a transmission image of the internal structure of a radiography target is obtained on the basis of the amount of X-ray attenuation accumulated during the process of penetrating the radiography target. To obtain such an X-ray image, an X-ray image radiographing apparatus includes a generator for emitting X-rays to a radiography target, a detector for detecting X-rays that have passed the radiography target, and an image processor for obtaining an X-ray image of the radiography target as a detection result of the detector.

Recently, X-ray radiography has been rapidly replaced by digital radiography (DR) using a digital detector, driven by rapid development in semiconductor and information processing technologies, and radiography methods have also been developed in various ways depending on the purpose or application.

For example, an X-ray panorama image, which is mainly used in the dental field, displays a region of interest on a curved surface, that is, the shape of the dentition along dental arch trajectories, onto a plane. To obtain such an X-ray panorama image, an X-ray panorama radiography apparatus linearly moves and rotates a generator and a detector, which face each other with a radiography target therebetween, about a rotation axis between the generator and the detector by a particular angle to obtain 2D X-ray frame images for respective sections of dental arch trajectories, and reconstructs these 2D X-ray frame images using a predetermined panorama reconstruction algorithm, thereby obtaining an X-ray panorama image. For reference, an X-ray panorama image is known to be one of the most widely used X-ray images in dentistry, as it allows easy identification of the overall arrangement of teeth for the dental arch trajectories with a single image.

As another example, an X-ray computed tomography image (X-ray CT image) represents a 3D volume image of a radiography target positioned within the field of view (FOV) in the cylindrical shape. To obtain such an X-ray CT image, a CT radiography apparatus rotates a generator and a detector, which face each other with a radiography target therebetween, about a rotation axis between the generator and the detector by a particular angle to obtain 2D X-ray frame images in multiple directions of the radiography target, and reconstructs these 2D X-ray frame images using a predetermined CT algorithm, thereby obtaining an X-ray CT image. For reference, an X-ray CT image is widely used in cases where accurate information on the internal structure of a radiography target is required, such as implant procedures, as the X-ray CT image can represent a 3D volume image of a radiography target composed of voxels as well as cross-sectional images at desired positions and directions based on the 3D volume image.

A general dental X-ray image radiographing apparatus includes a column-shaped support vertically installed on the floor, a lift moving up and down along the support, a gantry installed at the lift, and a generator and a sensor installed at the opposite ends of the gantry with respect to a rotation axis. When the height of the gantry is adjusted according to a subject's height, the subject is positioned between the generator and the sensor. As the gantry rotates about the rotation axis, the generator and the sensor obtain 2D X-ray frame images for acquiring an X-ray panorama image or an X-ray CT image of the subject. The 2D X-ray frame images can ultimately be reconstructed into the X-ray panorama image or the X-ray CT image through image processing.

Such a dental X-ray image radiographing apparatus uses X-rays in the form of a cone beam because the generator and the sensor rotate at fixed heights to radiograph a subject. As a result, magnification varies depending on the position of the subject within the X-ray image, and distortions such as cone beam artifacts may occur.

The technology behind the present disclosure is disclosed in Korean Patent No. 10-2665771.

The foregoing is intended merely to aid in the understanding of the background of the present disclosure, and is not intended to mean that the present disclosure falls within the purview of the related art that is already known to those skilled in the art.

### SUMMARY OF THE INVENTION

Accordingly, the present disclosure has been made keeping in mind the above problems occurring in the related art, and the present disclosure is directed to providing a dental X-ray radiography apparatus and a method of operating the same, the apparatus and the method being capable of obtaining X-ray images with high image quality by adopting a spiral radiography method using fan beam X-rays, compared to a conventional dental X-ray radiography apparatus using cone beams.

However, technical objectives that the embodiment of the present disclosure is intended to achieve are not limited to the above-described technical objectives, and there may be other technical objectives.

As a technical means for realizing the technical objective of the present disclosure, according to an embodiment of the present disclosure, there is provided an X-ray radiography apparatus including: a radiography part including an X-ray generator and an X-ray sensor facing each other with a radiography target therebetween; a first driving part configured to raise or lower the radiography part from a first position to a second position; and a second driving part configured to rotate the X-ray generator and the X-ray sensor while the X-ray generator and the X-ray sensor face each other, wherein the second driving part is configured to sequentially increase a rotation speed of the X-ray generator and the X-ray sensor, maintain the rotation speed at a constant speed, and decrease the rotation speed when the radiography part is raised or lowered from the first position to the second position by the first driving part.

The above-mentioned solutions are merely exemplary and should not be construed as limiting the present disclosure. In addition to the above-described exemplary embodiment, additional embodiments may exist in the drawings and detailed description of the disclosure.

According to the above-described solution of the present disclosure, X-ray images with high image quality can be obtained by adopting a spiral CT method, compared to a conventional dental X-ray radiography apparatus using a cone beam CT method.

According to the above-described solution of the present disclosure, the radiography part is lowered to a subject's shoulder area according to the subject's height and then raised, thereby preventing contact between the subject's shoulders and the radiography part, which is lowered downward during the radiography process of the X-ray image.

However, effects achieved by the present disclosure are not limited to the above-described effects, and there may be other effects.

### BRIEF DESCRIPTION OF THE DRAWINGS

The above and other objectives, features, and other advantages of the present disclosure will be more clearly understood from the following detailed description when taken in conjunction with the accompanying drawings, in which:
FIG. 1 is a schematic configuration diagram illustrating a dental X-ray radiography apparatus according to an embodiment of the present disclosure;
FIG. 2 is a schematic block diagram illustrating a dental X-ray radiography apparatus according to an embodiment of the present disclosure;
FIGS. 3A and 3B are diagrams illustrating a radiography part raised from a first position to a second position, according to an embodiment of the present disclosure;
FIGS. 4A and 4B are diagrams illustrating a first position that varies with a subject's height, according to an embodiment of the present disclosure;
FIG. 5 is a schematic configuration diagram illustrating a radiography part according to an embodiment of the present disclosure; and
FIG. 6 is an operation flowchart illustrating a method of operating a dental X-ray radiography apparatus according to an embodiment of the present disclosure.

### DETAILED DESCRIPTION OF THE INVENTION

Hereinbelow, an exemplary embodiment of the present disclosure will be described in detail with reference to the accompanying drawings such that the present disclosure can be easily embodied by those skilled in the art to which this present disclosure belongs. However, the present disclosure may be embodied in various different forms and should not be limited to the embodiment set forth herein. Further, in order to clearly explain the present disclosure, portions that are not related to the present disclosure are omitted in the drawings, and like reference numerals designate like elements throughout the specification.

Throughout the specification of the present disclosure, when a part is referred to as being "connected" to another part, it includes not only being "directly connected", but also being "electrically connected" or "indirectly connected".

Throughout the specification, when a member is said to be "on", "at an upper portion of", "on top of", "under", "at a lower portion of", "at the bottom of" another member, this includes not only when the two members are in contact, but also when there is an intervening member between the two members.

Throughout the specification, when a part "includes" an element, it is noted that it further includes other elements, but does not exclude other elements, unless specifically stated otherwise.

The present disclosure relates to a dental X-ray radiography apparatus and a method of operating the same.

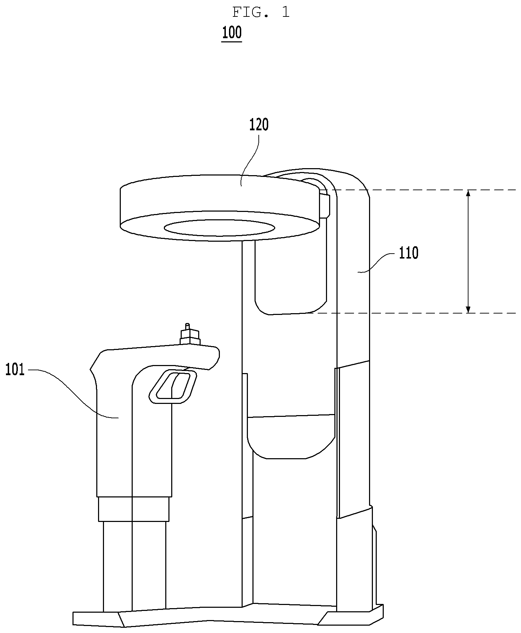

FIG. 1 is a schematic configuration diagram illustrating a dental X-ray radiography apparatus according to an embodiment of the present disclosure.

A dental X-ray radiography apparatus 100 (hereinafter, referred to as the X-ray radiography apparatus 100 according to an embodiment of the present disclosure is for spiral-type X-ray radiography using conventional cone beam X-rays or fan beam X-rays, and may include a support unit 110 and a radiography part 120 connected to one side of the support unit 110.

Referring to FIG. 1, the support unit 110 may be a column member vertically installed on the floor. In addition, referring to FIG. 1, the radiography part 120 is connected to one side of the support unit 110 and makes upward-downward movement, that is, being raised and lowered, along the support unit 110, and may have the shape of an O-ring with a hole in the center. In addition, the radiography part 120 may be configured as a gantry structure such that an X-ray generator 140 and an X-ray sensor 150 may rotate, facing each other with a radiography target therebetween.

When a subject 1 or 1' holds a handle frame 101 and positions his or her head within the radiography part 120, the X-ray radiography apparatus 100 allows the radiography part 120 to take upward-downward movement along the extension direction of the support unit 110, and rotates the X-ray generator 140 and the X-ray sensor 150, which are installed at the inner side the radiography part 120, with the radiography part 120 to obtain an X-ray image of the subject 1 or 1'.

Herein, the X-ray radiography apparatus 100 may generate high-resolution images compared to conventional methods, by using a cone beam method in which the generator and the sensor at positions fixed by a gantry rotate within an angle range limited to be less than 360 degrees and X-ray images are obtained with X-rays in the form of a cone beam, or by using a spiral method in which the X-ray generator 140 and the X-ray sensor 150 rotate and make upward-downward movement and the portion of the subject 1 or 1' to be radiographed are scanned in a spiral manner with X-rays in the form of a fan beam.

Hereinafter, an X-ray image radiographing process using the X-ray radiography apparatus 100 according to an embodiment of the present disclosure will be described in detail.

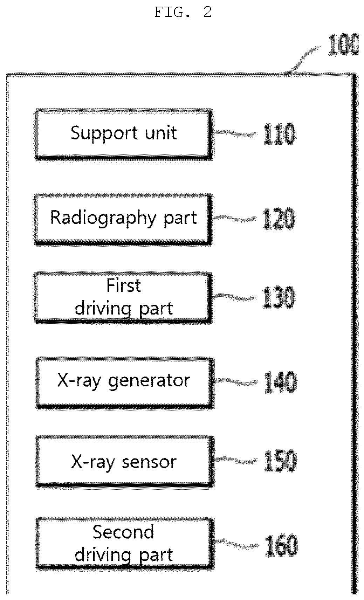

FIG. 2 is a schematic block diagram illustrating a dental X-ray radiography apparatus according to an embodiment of the present disclosure.

Referring to FIG. 2, the X-ray radiography apparatus 100 may include the support unit 110, the radiography part 120, a first driving part 130, the X-ray generator 140, the X-ray sensor 150, and a second driving part 160.

As described above, the support unit 110 may be a column member vertically installed on the floor. In addition, the radiography part 120 is connected to one side of the support unit 110 and makes upward-downward movement along the support unit 110, and may have the shape of an O-ring with a hole in the center to provide an alignment space of the radiography target. Herein, the radiography target may be the subject's head.

According to an embodiment of the present disclosure, the first driving part 130 may raise or lower the radiography part 120 from the first position to the second position along the extension direction of the support unit 110. As an example, when the first position is the lowest position along the movement path of the radiography part 120 and the second position is a higher position than the first position, the first driving part 130 may raise the radiography part 120 from the first position to the second position, which is the higher position than the first position. Herein, the movement path may refer to the path along which the radiography part 120 may be moved, and may indicate the path from the first position to the second position.

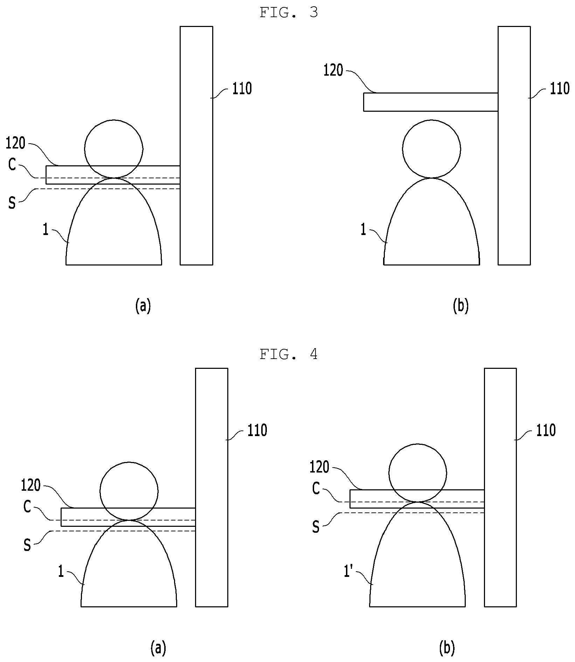

FIGS. 3A and 3B are diagrams illustrating a radiography part raised from a first position to a second position, according to an embodiment of the present disclosure.

Specifically, FIG. 3A may show the state in which the radiography part 120 is positioned at the lower of the first position and the second position, according to an embodiment of the present disclosure. In addition, FIG. 3B may show the state in which the radiography part 120 is positioned at the higher of the first position and the second position, according to an embodiment of the present disclosure.

Referring to FIG. 3A, at the lower of the first position and the second position, at least a portion, for example, the bottom surface, of the radiography part 120 may be positioned higher than the shoulders (S) and lower than the chin (C) of the subject 1 or 1'. In this regard, the lower of the first position and the second position may be the lowest position along the movement path of the radiography part 120, and may be the position at which the bottom surface of the radiography part 120 does not come into contact with the shoulders of the subject 1 or 1'. In addition, the inner diameter of the radiography part 120 may be smaller than the shoulder width of the subject 1 or 1'. This may be because, as the X-ray radiography apparatus 100 according to an embodiment of the present disclosure is a dental apparatus and the radiography range is limited to the head of the subject 1 or 1', the radiography part 120 does not need to be lower than the shoulders of the subject 1, 1'.

Referring to FIG. 3B, at the higher of the first position and the second position, at least a portion, for example, the top surface, of the radiography part 120 may be positioned higher than the head of the subject 1 or 1'. Specifically, the radiography range of the X-ray radiography apparatus 100 according to an embodiment of the present disclosure may be limited to the head of the subject 1 or 1'**.** However, the skeletal structure of the head may vary depending on the individual. Therefore, in order to obtain an image covering the entire radiography range, the second position may be a position at which at least the top surface of the radiography part 120 is above the head of the subject 1 or 1'.

According to an embodiment of the present disclosure, the first driving part 130 may lower the radiography part 120 to the lowest position at which the radiography part 120 does not come into contact with the shoulders of the subject 1 or 1', when the subject 1 or 1' is positioned at a center region within the radiography part 120 and preparation for radiographying an X-ray image by an operator is completed.

Herein, for example, when the first position is lower than the second position, at least one sensor for measuring the distance between the shoulder of the subject 1 or 1' and the radiography part 120 is installed at the bottom surface of the radiography part 120, and the first driving part 130 may determine the lowest position, that is, the first position, along the movement path of the radiography part 120 on the basis of the distance between the shoulder of the subject 1 or 1' and the radiography part 120 measured by the at least one sensor. However, without being limited thereto, the first position may be set by the operator directly driving the first driving part 130 while checking the shoulder positions of the the subject 1 or 1'. To this end, the lower surface of the radiography part 120 may be provided with a horizontal beam unit for emitting a laser at a height equal to or lower than the lower surface of the radiography part 120. The operator may place the radiography part 120 at the first position while checking whether the laser from the horizontal beam unit is emitted to the shoulders of the subject 1 or 1'.

FIGS. 4A and 4B are diagrams illustrating a first position that varies with a subject's height, according to an embodiment of the present disclosure. Hereinafter, for convenience of description, the first position will be specified as a position relatively lower than the second position, that is, a position at which the radiography part 120 is positioned under the chin of the subject 1 or 1'. That is, the first position described in FIGS. 4A and 4B may be a position at a height equal to or higher than the shoulders and equal to or lower than the chin of the subject 1 or 1', along the movement path of the radiography part 120.

Referring to FIGS. 4A and 4B, it can be seen that the height of a first subject 1 shown in FIG. 4A and the height of a second subject 1' shown in FIG. 4B are different, resulting in different first positions at which the radiography part 120 may be positioned. In other words, the first position may be variable depending on the height of the subject 1 or 1'.

In addition, the second position may also be variable, accordingly. However, without being limited thereto, the second position may be set to a sufficiently high position applicable to subjects' various heights, or may be a position corresponding to the highest position of the radiography part 120, that is, the maximum height of the support unit 110, allowed in the X-ray radiography apparatus 100 according to an embodiment of the present disclosure in order to secure as much projection data as possible while the radiography part 120 is raised.

According to an embodiment of the present disclosure, the second driving part 160 may start rotating the X-ray generator 140 and the X-ray sensor 150 installed at the inner side of the radiography part 120 when the first driving part 130 has stopped the radiography part 120 at the first position.

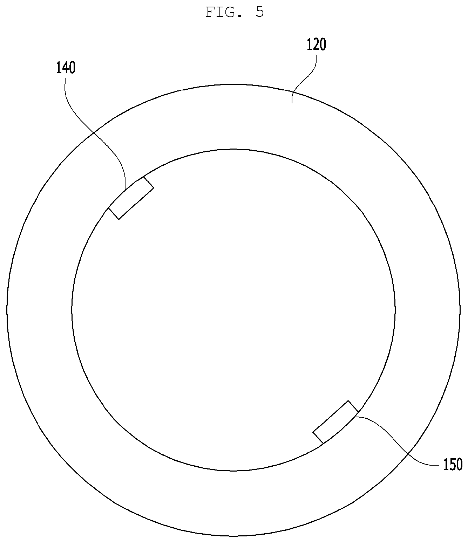

FIG. 5 is a schematic configuration diagram illustrating a radiography part according to an embodiment of the present disclosure.

Referring to FIG. 5, the X-ray generator 140 and the X-ray sensor 150 may be installed at the inner side of the radiography part 120. Herein, the X-ray generator 140 and the X-ray sensor 150 may be installed facing each other.

In this regard, the X-ray generator 140 may generate X-rays toward a radiography target portion corresponding to the head of the subject 1 or 1', and the X-ray sensor 150 may detect X-rays that have passed through the radiography target portion. The X-ray radiography apparatus 100 according to an embodiment of the present disclosure may obtain 2D image data, that is, the projection data, of the radiography target portion from multiple angles using the X-ray generator 140 and the X-ray sensor 150.

In addition, according to an embodiment of the present disclosure, the second driving part 160 may rotate the X-ray generator 140 and the X-ray sensor 150 according to the shape of the radiography part 120. As described above, the second driving part 160 may start rotating the X-ray generator 140 and the X-ray sensor 150 when the radiography part 120 is stopped at the first position.

According to an embodiment of the present disclosure, in accordance with the upward-downward movement of the radiography part 120 by the first driving part 130, that is, when the radiography part 120 is raised or lowered, the second driving part 160 may sequentially perform the following: increase the rotation speed of the X-ray generator 140 and the X-ray sensor 150, maintain the rotation speed of the X-ray generator 140 and the X-ray sensor 150 at a constant speed, and decrease the rotation speed of the X-ray generator 140 and the X-ray sensor 150.

Specifically, when the radiography part 120 is stopped at the first position, the second driving part 160 may start rotating the X-ray generator 140 and the X-ray sensor 150 and gradually increase the rotation speed. In other words, the second driving part 160 may increase the rotation speed of the X-ray generator 140 and the X-ray sensor 150 while the radiography part 120 is placed at the first position.

Herein, the X-ray generator 140 and the X-ray sensor 150 may obtain an auxiliary X-ray image when the rotation speed is increased, that is, when the radiography part 120 is stopped at the first position. Herein, the auxiliary X-ray image may be used to correct a truncation artifact, that is, an artifact caused by truncation at the lower end of the field of view (FOV), in the X-ray image obtained as the first driving part 130 starts raising the radiography part 120 from the first position and, accordingly, the second driving part 160 rotates the X-ray generator 140 and the X-ray sensor 150 at a constant speed.

In addition, the second driving part 160 may increase the rotation speed of the X-ray generator 140 and the X-ray sensor 150, and may maintain the rotation speed at a constant speed when a predetermined speed is reached. Herein, the first driving part 130 may raise the radiography part 120 from the first position to the second position when the rotation speed of the X-ray generator 140 and the X-ray sensor 150 reaches the predetermined speed and enters a constant-speed state. In addition, the second driving part 160 may maintain the rotation speed at a constant speed until the radiography part 120 reaches the second position when the first driving part 130 raises the radiography part 120 from the first position to the second position.

Herein, the X-ray generator 140 and the X-ray sensor 150 may be raised and rotated by the first driving part 130 and the second driving part 160, and may thus be ascended while rotated in a spiral manner to obtain an X-ray image of the FOV.

In addition, the second driving part 160 may decrease the rotation speed of the X-ray generator 140 and the X-ray sensor 150 when the radiography part 120 reaches the second position, that is, when the X-ray generator 140 and the X-ray sensor 150 are out of the field of view (FOV), which is an area to be radiographed. In other words, the second driving part 160 may reduce the rotation speed of the X-ray generator 140 and the X-ray sensor 150 while the radiography part 120 is placed at the second position.

Specifically, the first driving part 130 may raise the radiography part 120 to the second position, and may stop the radiography part 120 at the second position when the radiography part 120 reaches the second position. However, no limitation thereto is imposed. For example, the first driving part 130 may raise the radiography part 120 to a predetermined position higher than the second position. This may be intended to secure sufficient temporal and spatial margins for decreasing the rotation speed of the X-ray generator 140 and the X-ray sensor 150 after securing projection data of the FOV, and to prevent a problem in which the deceleration of the X-ray generator 140 and the X-ray sensor 150 starts too early and the sufficient acquisition of the projection data of the FOV may not be guaranteed.

In this regard, the X-ray radiography apparatus 100 according to an embodiment of the present disclosure may secure a sufficient deceleration phase at the height equal to or higher than the second position, which is above a subject's head, by obtaining an X-ray image while raising the radiography part 120 from the first position to the second position. Therefore, compared to the case in which an X-ray image is obtained by lowering the radiography part 120 from the second position to the first position, omission or truncation of the projection data of the FOV may be prevented.

In other words, the second driving part 160 may sequentially increase, maintain at a constant speed, and decrease the rotation speed when the radiography part 120 is moved from the first position to the second position. In the acceleration phase in which the rotation speed is increased, the radiography part 120 may be stopped at the first position. In the constant-speed phase in which the rotation speed is at a constant speed, the radiography part 120 may be raised from the first position to the second position. In the deceleration phase in which the rotation speed is decreased, the radiography part 120 may be stopped at the second position or may be raised to a predetermined height above the second position.

In addition, the X-ray generator 140 and the X-ray sensor 150 obtain an auxiliary X-ray image in the acceleration phase, obtain an X-ray image in the constant-speed phase, and end radiographying in the deceleration phase.

However, without being limited thereto, radiographying a radiography target by the radiography part 120 may be performed while the radiography part 120 is lowered. That is, according to an embodiment of the present disclosure, the first driving part 130 may place the radiography part 120 at the second position, that is, a relatively higher position, and the second driving part 160 may increase the rotation speed of the X-ray generator 140 and the X-ray sensor 150 while the radiography part 120 is placed at the second position. In addition, when the first driving part 13 lowers the radiography part 120 from the second position to the first position, that is, a relatively lower position, the second driving part 130 may rotate the X-ray generator 140 and the X-ray sensor 150 at a constant speed. In addition, the first driving part 130 may lower the radiography part 120 to the first position to place the radiography part 120 at the first position, and the second driving part 160 may decrease the rotation speed of the X-ray generator 140 and the X-ray sensor 150 while the radiography part 120 is placed at the first position.

As described above, the X-ray radiography apparatus 100 may obtain X-ray images with high image quality by adopting a spiral CT method, compared to a conventional dental X-ray radiography apparatus using a cone beam CT method.

In addition, the X-ray radiography apparatus 100 lowers the radiography part 120 to a subject's shoulder area according to the subject's height and then raises the radiography part, thereby preventing contact between the subject's shoulders and the radiography part 120, which is lowered downward during the radiography process of the X-ray image.

Hereinafter, based on the details described above, the operation flow of the present disclosure will be simply described.

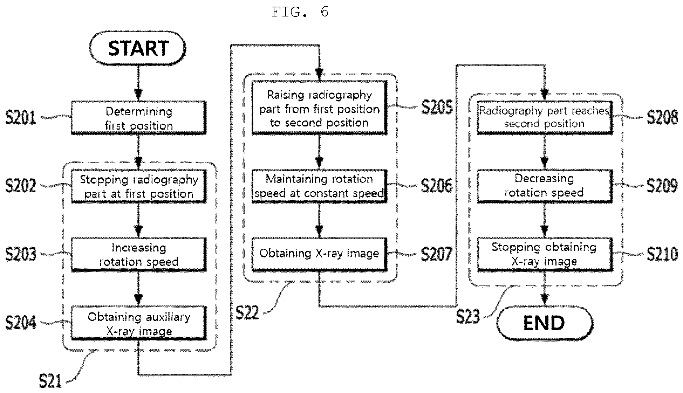

FIG. 6 is an operation flowchart illustrating a method of operating a dental X-ray radiography apparatus according to an embodiment of the present disclosure.

The method of operating the dental X-ray radiography apparatus shown in FIG. 6 may be performed by the dental X-ray radiography apparatus 100 described above. Accordingly, even if a particular description is omitted below, the description provided for the dental X-ray radiography apparatus 100 may be equally applicable to the description of the method of operating the dental X-ray radiography apparatus.

Referring to FIG. 6, in step S201, the first position may be determined according to a subject's height.

In step S202, the first driving part 130 may stop the radiography part 120 at the lower of the first position and the second position.

In step S203, the second driving part 160 may increase the rotation speed of the X-ray generator 140 and the X-ray sensor 150.

In step S204, the X-ray generator 140 and the X-ray sensor 150 may obtain an auxiliary X-ray image.

Herein, steps S202 to S204 may be performed simultaneously or in a partially different order in terms of sequence. The step S21 in which the X-ray generator 140 and the X-ray sensor 150 are rotated with increasing speed and obtain the auxiliary X-ray image while the radiography part 120 is placed at the lower of the first position and the second position may be referred to as the acceleration phase.

Next, in step S205, the first driving part 130 may raise the radiography part 120 from the lower of the first position and the second position to the higher of the first position and the second position.

Next, in step S206, the second driving part 160 may maintain the rotation speed of the X-ray generator 140 and the X-ray sensor 150 at a constant speed.

Next, in step S207, the X-ray generator 140 and the X-ray sensor 150 may obtain an X-ray image.

Herein, steps S205 to S207 may be performed simultaneously or in a partially different order in terms of sequence. The step S22 in which the X-ray generator 140 and the X-ray sensor 150 are rotated at a constant speed and obtain the X-ray image while the radiography part 120 is raised from the lower of the first position and the second position to the higher of the first position and the second position may be referred to as the constant-speed state.

Next, in step S208, the first driving part 130 may move the radiography part 120 such that the radiography part reaches the second position.

Next, in step S209, the second driving part 160 may decrease the rotation speed of the X-ray generator 140 and the X-ray sensor 150.

Next, in step S210, the X-ray generator 140 and the X-ray sensor 150 may stop obtaining the X-ray image.

Herein, steps S208 to S210 may be performed simultaneously or in a partially different order in terms of sequence. The step S23 in which the X-ray generator 140 and the X-ray sensor 150 are rotated with decreasing speed and obtain the auxiliary X-ray image while the radiography part 120 is placed at the higher of the first position and the second position may be referred to as the deacceleration phase.

In the above description, steps S21 to S23, S201, and S210 may be further divided into additional steps or combined into fewer steps according to embodiments of the present disclosure. In addition, some steps may be omitted when necessary, and the order of the steps may be changed.

A method of operating a dental X-ray radiography apparatus according to an embodiment of the present disclosure may be implemented as program instructions executable by various computer means and may be recorded on a computer-readable medium. The computer-readable recording medium may include program commands, data files, data structures, and the like separately or in combinations. The program commands to be recorded on the computer-readable recording medium may be specially designed and configured for embodiments of the present disclosure or may be well-known to and be usable by those skilled in the art of computer software. Examples of the computer-readable recording medium include magnetic recording media such as hard disks, floppy disks and magnetic tapes; optical data storage media such as CD-ROMs or DVD-ROMs; magneto-optical media such as floptical disks; and hardware apparatuss, such as read-only memory (ROM), random-access memory (RAM), and flash memory, which are particularly structured to store and implement the program instruction. Examples of the program instructions include not only a mechanical language code formatted by a compiler but also a high level language code that may be implemented by a computer using an interpreter, and the like. The hardware apparatuss may be configured to be operated by one or more software modules or vice versa to conduct the operation according to the present disclosure.

In addition, the method of operating the dental X-ray radiography apparatus described above may also be implemented in the form of a computer program or application executed by a computer and stored on a recording medium.

The above description of the present disclosure is for illustrative purposes only, and those skilled in the art to which the present disclosure pertains will understand that the present disclosure can be embodied in other specific forms without changing the technical ideas or essential characteristics of the present disclosure. Therefore, it should be understood that the embodiments described above are illustrative in all aspects and not restrictive. For example, each element described in a singular form can also be implemented in a distributed manner. Likewise, elements described as being distributed can be implemented in a combined form.

The scope of the present disclosure is defined by the appended claims rather than by the detailed description above. It shall be understood that all alterations or modifications derived from the meaning and scope of the claims and their equivalents are included in the scope of the present disclosure.

## Claims

1. An X-ray radiography apparatus, comprising:
a radiography part(120) including an X-ray generator(140) and an X-ray sensor(150) facing each other with a radiography target therebetween;
a first driving part(130) configured to raise or lower the radiography part (120) from a first position to a second position; and
a second driving part(160) configured to rotate the X-ray generator(140) and the X-ray sensor(150) while the X-ray generator(140) and the X-ray sensor(150) face each other,
**characterized in that** the second driving part(160) is configured to sequentially increase a rotation speed of the X-ray generator(140) and the X-ray sensor(150), maintain the rotation speed at a constant speed, and decrease the rotation speed when the radiography part(120) is raised or lowered from the first position to the second position by the first driving part(130).

2. The X-ray radiography apparatus of claim 1, wherein the radiography target is a subject's head,
at the lower of the first position and the second position, at least a portion of the radiography part(120) is positioned at a height equal to or higher than the subject's shoulders and equal to or lower than the subject's chin, and
at the higher of the first position and the second position, at least a portion of the radiography part(120) is positioned at a height equal to or higher than the subject's head.

3. The X-ray radiography apparatus of claim 1, wherein the second driving part(160) is configured to increase the rotation speed of the X-ray generator (140) and the X-ray sensor(150) while the radiography part(120) is placed at the lower of the first position and the second position.

4. The X-ray radiography apparatus of claim 1, wherein the second driving part(160) is configured to maintain the rotation speed of the X-ray generator(140) and the X-ray sensor(150) at the constant speed when the radiography part(120) is moved from the first position to the second position.

5. The X-ray radiography apparatus of claim 1, wherein the second driving part(160) is configured to decrease the rotation speed of the X-ray generator (140) and the X-ray sensor(150) while the radiography part(120) is placed at the higher of the first position and the second position.

6. The X-ray radiography apparatus of claim 1, wherein the X-ray generator(140) and the X-ray sensor(150) are configured to obtain an X-ray image when the rotation speed is at the constant speed.

7. The X-ray radiography apparatus of claim 1, wherein either the first position or the second position or both vary with the radiography target's height.
